# EUROPÄISCHE PATENTANMELDUNG

(11) **EP 2 149 572 A1**
(43) Veröffentlichungstag der Anmeldung: **03.02.2010**
(21) Anmeldenummer: 08450106.3
(22) Anmeldetag: 15.07.2008
(51) Int. Cl.: C07D 405/12, C08G 18/20

(54) **Melaminepoxide**

(71) Anmelder: Johannes Kepler Universität Linz, 4040 Linz (AT)
(72) Erfinder: Schwarzinger, Clemens, PD DI Dr., 4600 Wels (AT); Leidl, Manuela, DI. Dr., 5282 Ranshofen (AT); Schütz, Irene, 4113 St. Martin i. Mk. (AT)
(74) Vertreter: Bachinger-Fuchs, Eva-Maria

(57) **Zusammenfassung**

Die Erfindung betrifft Melaminepoxid der allgemeinen Formel I 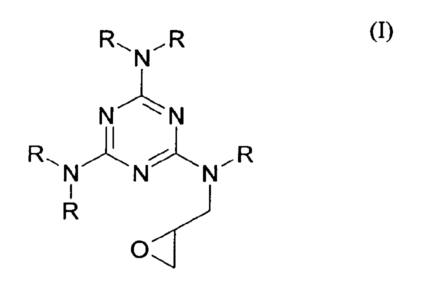 wobei R unabhängig aus der Gruppe, bestehend aus H, (C₁-C₆)-Alkyl, Allyl und Glycidyl, ausgewählt ist. Diese Verbindungen können zur Bildung von Epoxidharzen mit bekannter Vemetzungsdichte verwendet werden.

## Beschreibung

Die Erfindung betrifft Melaminepoxide der allgemeinen Formel I, daraus erhältliche Epoxidharze sowie Verfahren zur Herstellung der Melaminepoxide.

Epoxidharze auf der Basis von Phenolen (Bisphenol A od F, sowie Phenol-Formaldehyd-Kondensaten) sind seit ca. 1950 bekannt und in Patenten beschrieben (z.B. US 837,745).

Die Epoxidierung wird derart durchgeführt, dass die phenolische Komponente im zehnfachen Überschuss an Epichlorhydrin gelöst und auf 80°C erwärmt wird, wonach NaOH zum Abfangen der entstehenden HCl portionsweise zugegeben wird. Nach erfolgter Reaktion wird überschüssiges Epichlorhydrin abdestilliert und das Harz in Benzol aufgenommen, worauf das entstandene Salz ausfällt. Durch Zugabe von Phenol vor der Reaktion kann die Viskosität des Harzes erniedrigt werden.

In der EP 0 087 311 A sind Klebstoff-Formulierungen beschrieben, die als einen von 3 Bestandteilen N-Glycidyl-Komponenten enthalten. Derivate des Melamins werden darin nicht erwähnt.

Für Klebstoffe und Vernetzer von Lacken sind Epoxidharze von Interesse, die eine thermische und chemische Stabilität aufweisen und eine einstellbare Anzahl von Epoxidgruppen besitzen. Es besteht insbesondere sowohl Bedarf an Produkten, die flüssig sind, als auch an solchen, die von nicht zu niedriger Viskosität oder fest sind.

Die Erfindung stellt sich die Aufgabe, Epoxidverbindungen bereitzustellen, die zu Epoxidharzen führen, welche die oben genannten Anforderungen erfüllen. Insbesondere soll bei den bereitgestellten Verbindungen die Anzahl an Epoxidgruppen bzw. Glycidylresten steuerbar und damit die Vernetzungsdichte des Harzes im vorhinein festlegbar sein.

Diese Aufgabe wird erfindungsgemäß durch Melaminepoxid der allgemeinen Formel I gelöst, 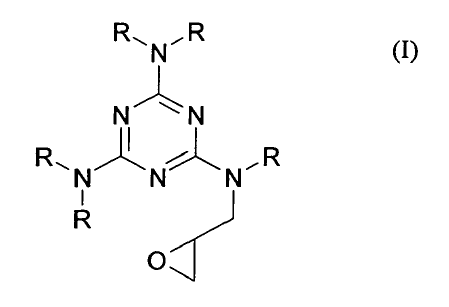 wobei R unabhängig aus der Gruppe, bestehend aus H, (C₁-C₆)-Alkyl, Allyl und Glycidyl, ausgewählt ist.

Ein weiterer Aspekt der Erfindung betrifft Epoxidharz, das durch Polymerisation (Polyaddition) von Melaminepoxiden der allgemeinen Formel I erhältlich ist und sich durch die folgende allgemeine Formel X darstellen lässt (wobei R dieselbe Bedeutung wie oben hat):

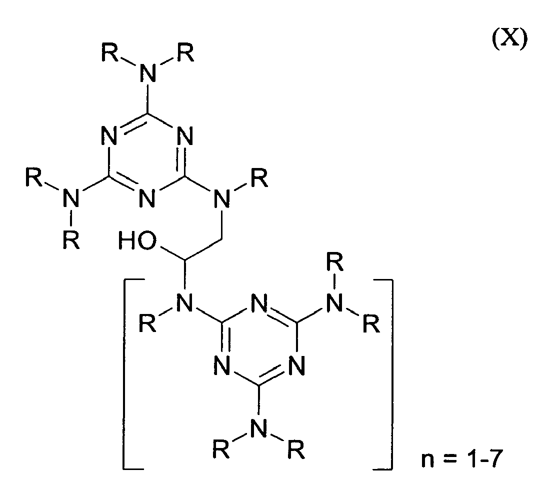

Die erfindungsgemäßen Melaminderivate können durch das Vorhandensein mehrerer Epoxidgruppen eine höhere Vemetzungsdichte aufbauen als z.B. Bisphenol-Epoxidharze, was für eine Anwendung des Epoxidharzes als Vernetzer in Lacksystemen vorteilhaft ist.

Die Erfindung betrifft weiters Verfahren zur Herstellung der Melaminepoxide der allgemeinen Formel I.

Eines dieser Verfahren ist **dadurch gekennzeichnet, dass** Alkylmelamin der Formel II 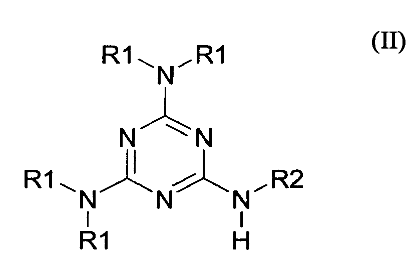 wobei
R1 unabhängig ausgewählt ist aus der Gruppe bestehend aus H und (C₁-C₆)-Alkyl, und
R2 (C₁-C₆)-Alkyl ist,
in Gegenwart eines Alkalihydroxids, vorzugsweise NaOH oder KOH, mit Epichlorhydrin oder Epibromhydrin umgesetzt wird.

Bei diesem Verfahren werden Alkylmelamine als Ausgangsverbindungen gewählt. Diese weisen durch die Alkylgruppen überraschenderweise eine erhöhte Reaktivität an den Amino/Iminogruppen auf. Außerdem wird durch die Substitution des Melamins mit Alkylgruppen eine verbesserte Löslichkeit in Epichlorhydrin erreicht, was für die Durchfiihrbarkeit der Reaktion in technologischer Hinsicht entscheidend ist. Als Nebenprodukte können auch O-Glycidylverbindungen entstehen.

Das Verfahren wird vorzugsweise bei Normaldruck und Temperaturen von 50°C-150°C durchgeführt. Bei erhöhtem Druck im Autoklaven ist die Umsetzung ebenso möglich. Die Reaktion findet ansatzweise aber auch bei Raumtemperatur statt.

Ein alternatives Verfahren ist **dadurch gekennzeichnet, dass** Allylmelamin der Formel III 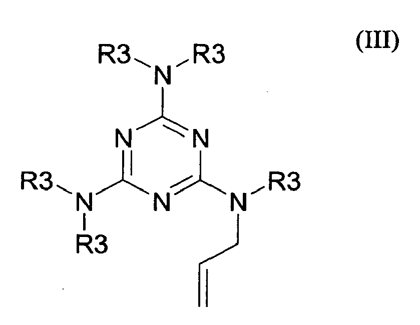 wobei R3 unabhängig ausgewählt ist aus der Gruppe bestehend aus H, (C₁-C₆)-Alkyl und Allyl, mit einem Oxidationsmittel umgesetzt wird.

Vorzugsweise wird das Oxidationsmittel aus der Gruppe, bestehend aus Natriumhypochlorit, Wasserstoffperoxid und Persäuren, z.B. Perbenzoesäure, ausgewählt.

Mittels dieser Verfahrensvariante gelingt selbst eine vollständige Substitution des Melamins mit Glycidylresten (Hexaglycidylmelamin).

Die Erfindung wird nachfolgend anhand von Beispielen näher erläutert.

### Beispiel 1

1,86 g 2,4,6-Trimethylmelamin wwerden in 10 ml Epichlorhydrin gelöst und mit 1,3 g NaOH (1:3 in Wasser) versetzt. Die Reaktionsmischung wird unter Rückfluss 2 Stunden gekocht und danach das überschüssige Epichlorhydrin im Vakuum abdestilliert. Das Produkt ist ein viskoses, hellgelbes Öl, das laut massenspektrometrischer Analyse folgende Komponenten enthält:
N-Glycidyl-trimethylmelamin (MH⁺=225)
N,N'-Diglycidyl-trimethylmelamin (MH⁺=281)
N,N',N"-Triglycidyl-trimethylmelamin (MH⁺=337)

Es entstehen Dimere mit unterschiedlichstem Substitutionsmuster sowie Chlorhydrine und Glykole.

HPLC/UV/MS Analyse:

| | |
|---|---|
| Säule: | Zorbax SB-C18 150x2,1 mm, 5µ |
| Gradient: | A ... 0,1% Ameisensäure in Wasser |
| | B ... Acetonitril |
| | von 100% A auf 50% A in 55 min; dann rückspülen auf 100% A und |
| | 10 min halten |
| Säulentemperatur: | 40°C |
| Injektion: | 30 µl |
| Detektion: | UV @ 254 nm |
| | ESI-MS m/z=80-1000 |

Eine vollständige Isolierung und Auftrennung der einzelnen glycidylsubstituierten Melaminderivate kann durch chromatographische Verfahren erreicht werden.

### Beispiel 2

1,01 g 2,2,4,6-Tetramethylmelamin werden in 10 ml Epichlorhydrin gelöst und mit 1,97 g NaOH (1:3 in Wasser) versetzt. Die Reaktionsmischung wird bei 95°C für 5 Stunden refluxiert und danach das überschüssige Epichlorhydrin im Vakuum abgezogen. Der Rückstand wird mit 1,99 g NaOH (1:3 in Wasser) versetzt und 30 Minuten lang ausgerührt.

Danach wird das Lösungsmittel erneut im Vakuum abdestilliert. Das Produkt ist gelb und von honigartiger Konsistenz und enthält die Monoglycidylverbindungen (MH⁺=239) und Diglycidylverbindungen (MH⁺=295) im Verhältnis 2:1 (bestimmt über ESI-MS).

### Beispiel 3

1,91 g 2,4,6-Trimethylmelamin und 2,92 g KOH (1:3 in Wasser) werden in 10 ml Epichlorhydrin gelöst und 1 Stunde bei 95°C refluxiert. Das überschüssige Epichlorhydrin wird im Vakuum abdestilliert. Als Produkt erhält man N-Glycidyl-2,4,6-Trimethylmelamin (MH⁺=225) sowie geringe Mengen an N, N'-Diglycidyl-2,4,6-Trimethylmelamin (MH⁺=281).

### Beispiel 4

100 mg Hexaallylmelamin werden in 5 ml Tetrahydrofuran gelöst und mit 10 ml Natriumhypochloritlösung gemischt. Die Lösung wird unter Rühren für 12 Stunden mit einer Badtemperatur von 80°C refluxiert. Nach Ende der Reaktion tritt eine Phasenseparation auf und die obere Phase wird im Vakuum zur Trockene eingedampft.
Analyse mit ESI Massenspektrometrie in Acetonitril/Wasser/Trifluoressigsäure zeigt folgende Produkte:
Hexaallylmelamin (MH⁺=367)
N-Glycidyl-pentaallylmelamin (MH⁺=383)
N,N'-Diglydicyl-tetraallylmelamin (MH⁺=399)
N-(2,3-dihydroxypropyl)-tetraallylmelamin (MH⁺=401)
N,N,N',N"-Tetraglycidyl-diallylmelamin (MH⁺=431)
Pentaglycidyl-allylmelamin (MH⁺=447)

### Beispiel 5

1 g Hexaallylmelamin wird in 5 ml Tetrahydrofuran gelöst und in eine Lösung von 4,3 g Chlorperbenzoesäure in Tetrahydrofuran langsam unter Kühlung auf 0°C zugetropft. Die Reaktion wird für 24 Stunden bei 0°C gehalten und die Reaktionsmischung danach im Vakuum zur Trockene eingedampft.
Analyse mit ESI Massenspektrometrie in Acetonitril/Wasser/Trifluoressigsäure zeigt folgende Produkte:
Hexaallylmelamin (MH⁺=367)
N-Glycidyl-pentaallylmelamin (MH⁺=383)
N,N'-Diglycidyl-tetraallylmelamin (MH⁺=399)
N,N,N',N"-Tetraglycidyl-diallylmelamin (MH⁺=431)
Pentaglycidyl-allylmelamin (MH⁺=447)
Hexaglycidylmelamin (MH⁺=463)

### Beispiel 6

100 mg Hexaallylmelamin werden in 5 ml Chloroform suspendiert und 10 ml 30%iger Wasserstoffperoxidlösung, enthaltend 4% Molybdänoxid, werden zugegeben. Die zweiphasige Mischung wird für 48 h heftig gerührt und die Chloroformphase anschließend bei vermindertem Druck einrotiert. Das Produkt wird mit ESI Massenspektrometrie in Acetonitril/Wasser/Trifluoressigsäure analysiert:
Hexaallylmelamin (MH⁺=367)
N-Glycidyl-pentaallylmelamin (MH⁺=383)
N,N'-Diglycidyl-tetraallylmelamin (MH⁺=399)
N,N',N"-Triglycidyl-triallylmelamin (MH⁺=415)

### Beispiel 7

1 g Produkt aus Beispiel 1 wird mit 1 g Methyltetrahydrophthalsäure als Härter versetzt und mechanisch gemischt. 2 Stahlplatten werden mit dieser Mischung zusammengefügt und für 15 Minuten bei 140°C in einem Trockenschrank gehärtet. Nach dem Erkalten hat sich eine feste Verbindung ausgebildet.

### Beispiel 8

1 g Produkt aus Beispiel 1 wird mit 1 g kommerziell erhältlichem 5 Minuten Epoxy-Härter (sulfidisch) gemischt und 2 Holzteile werden mit dieser Mischung verbunden. Nach 15 Minuten bildet sich eine feste Klebefuge.

## Patentansprüche

1. Melaminepoxid der allgemeinen Formel I 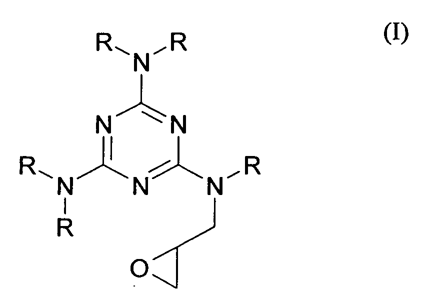 wobei R unabhängig aus der Gruppe, bestehend aus H, (C₁-C₆)-Alkyl, Allyl und Glycidyl, ausgewählt ist.

2. Epoxidharz, erhältlich durch Polymerisation von Melaminepoxiden der allgemeinen Formel I.

3. Verfahren zur Herstellung von Melaminepoxid der allgemeinen Formel I, **dadurch gekennzeichnet, dass** Alkylmelamin der Formel II 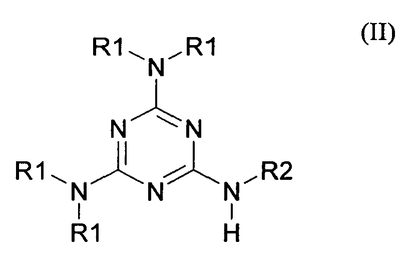 wobei
R1 unabhängig ausgewählt ist aus der Gruppe bestehend aus H und (C₁-C₆)-Alkyl, und
R2 (C₁-C₆)-Alkyl ist,
in Gegenwart eines Alkalihydroxids, vorzugsweise NaOH oder KOH, mit Epichlorhydrin oder Epibromhydrin umgesetzt wird.

4. Verfahren zur Herstellung von Melaminepoxid der allgemeinen Formel I, **dadurch gekennzeichnet, dass** Allylmelamin der Formel III 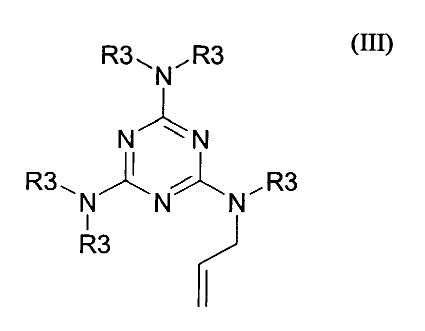 wobei R3 unabhängig ausgewählt ist aus der Gruppe bestehend aus H, (C₁-C₆)-Alkyl und Allyl, mit einem Oxidationsmittel umgesetzt wird.

5. Verfahren nach Anspruch 4, **dadurch gekennzeichnet, dass** das Oxidationsmittel aus der Gruppe, bestehend aus Natriumhypochlorit, Wasserstoffperoxid und Persäuren, ausgewählt wird.
